# EUROPÄISCHE PATENTSCHRIFT

(11) **EP 2 311 792 B1**
(45) Veröffentlichungstag und Bekanntmachung des Hinweises auf die Patenterteilung: **15.05.2013**
(21) Anmeldenummer: 09012899.2
(22) Anmeldetag: 13.10.2009
(51) Int. Cl.: C07C 201/12, C07C 205/45

(54) **Friedel-crafts-acylierung zur Synthese von Aryl- und Heteroaryl-(3-ethyl-4-nitro-phenyl)-Methanonen**
Friedel-Crafts acylation for synthesis of aryl and heteroaryl-(3-ethyl-4-nitro-phenyl)-methanones
Acylation de Friedel-Craft pour la synthèse d'aryl- et d'hétéroaryl-(3-éthyl-4-nitro-phényle)-méthanones

(43) Veröffentlichungstag der Anmeldung: 20.04.2011
(73) Patentinhaber: Roche Diagnostics GmbH, 68305 Mannheim (DE); F.Hoffmann-La Roche AG, 4070 Basel (CH)
(72) Erfinder: Knipp, Bernhard, 51515 Kuerten (DE)

(56) Entgegenhaltungen:
- WO-A2-2004/074300
- STEPHEN L. GOLDSTEIN AND EDWARD MCNELIS: "Migrations in Oxidations of Mesidine" JOURNAL OF ORGANIC CHEMISTRY, Bd. 49, 1984, Seiten 1613-1620, XP002577113

## Beschreibung

### Stand der Technik

Über die Licht-gesteuerte Synthese von hochverdichteten Oligonucleotid Microarrays unter Verwendung photolabiler 2-(2-Nitrophenyl)-propoxycarbonyl-Schutzgruppen (NPPOC) als 5'-O-carbonatester von Phosphoramidit-Bausteinen wurde verschiedentlich berichtet (Buehler et al., Nucleosides, Nucleotides & Nucleic Acids 24 (2005) 891; Helv. Chim. Acta 87 (2004) 620; WO 2004/074300). Die Synthese dieser Benzophenon-Schutzgruppe im Labormaßstab beginnt mit der Kupplung von Benzylcyaniden mit ortho-Nitroethylbenzol zum Cyano-Oxim und nachfolgender exothermer oxidativer Decarboxylierung durch Behandlung mit Wasserstoffperoxid (35%) in Kaliumhydroxid unter Sauerstoff-Evolution in kochendem Methanol Buehler et al., Nucleosides, Nucleotides & Nucleic Acids 24 (2005) 891; WO 2004/074300; Artini et al., Arzneim. Forsch. 21 (1971) 30). Alternativ-Synthesen zu Benzophenonen des Aryl- bzw. Heteroaryl-(3-ethyl-4-nitro-phenyl)-methanon Typs sind bislang unbekannt. Bis auf 3-Ethyl-4-nitro-benzophenon (s.u.) sind keine weiteren Aryl- bzw. Hetero-Analoga des 3-Ethyl-4-nitro-benzophenons als Stoff beschrieben.

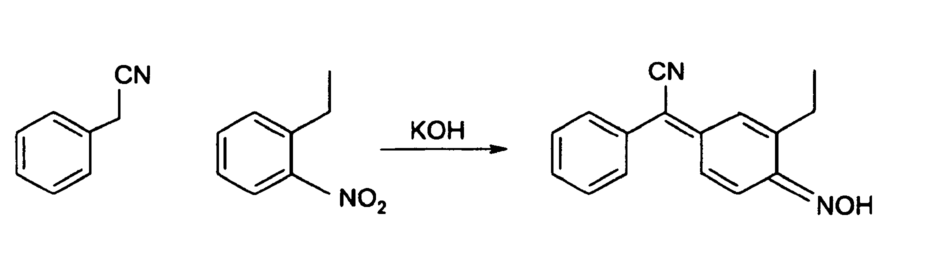

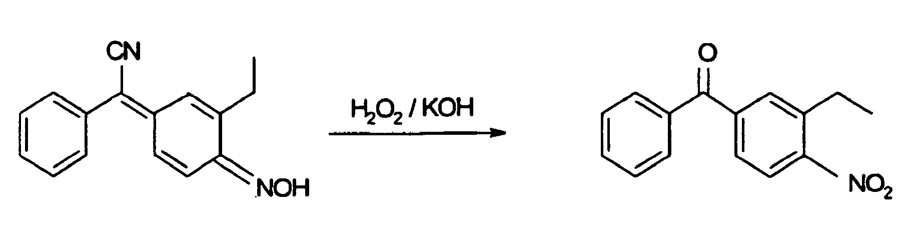

Die bisherige Herstellung erfolgt über zwei Stufen mit mäßigen Ausbeuten (ca. 26%) (WO 2004/074300). Die restlichen Reaktionsprodukte sind unbekannt (Buehler et al., Nucleosides, Nucleotides & Nucleic Acids 24 (2005) 891; WO 2004/074300; EP 1 589 024). Die Durchführung der oxidativen Decarboxylierung (s.o.) ist bezüglich Sicherheit und Umweltschutz im technischen Maßstab problematisch. Bei fehlender Inertisierung wegen ständiger Sauerstoff-Evolution im leichtflüchtigen und leichtentzündlichen Methanol (Buehler et al., Nucleosides, Nucleotides & Nucleic Acids 24 (2005) 891; WO 2004/074300; EP 1 589 024) sind potentiell Brände, Verpuffungen oder unter Umständen bei Bildung von Peroxiden aus Wasserstoffperoxid (s.o.) Explosionen zu befürchten. Außerdem ist bei unvollständiger oxidativer Decarboxylierung die Generierung von giftigen Cyaniden in Abwasser (KCN) und Abluft (HCN, Dicyan) zu bewerten. Der allgemeine synthetische Zugang ist per se beschränkt.

Die der vorliegenden Erfindung zugrundeliegende Aufgabe besteht somit in der Bereitstellung eines verbesserten Verfahrens zur Herstellung von Phosphoramiditen mit photolabiler NPPOC-Schutzgruppe und der Herstellung von neuen bisher nicht beschriebenen photolabilen NPPOC-Schutzgruppen als Phosphoramidit-Bausteine.

### Kurzbeschreibung der Erfindung

Gegenstand der vorliegenden Erfindung ist somit ein Syntheseverfahren enthaltend die folgenden Schritte
a) Umsetzung von 3-Ethyl-4-nitrobenzoesäure mit einem Thionylhalogenid (vorzugsweise Thionlychlorid) zur Erzeugung eines 3-Ethyl-4-nitrobenzoesäurehalogenids
b) Friedel-Crafts-Acylierung durch Reaktion des 3-Ethyl-4-nitrobenzoesäurehalogenids mit gegebenenfalls substituiertem Aryl-H, so dass ein gegebenenfalls substituiertes (3-Ethyl-4-nitrophenyl)-aryl-methanon entsteht.

Vorzugsweise handelt es sich bei Aryl-H um Benzol oder einen gegebenenfalls substituierten kondensierten Aromaten wie beispielsweise Naphthalin.

Erfindungsgemäß können die gegebenenfalls substituierten (3-Ethyl-4-nitrophenyl) aryl-methanone in einem weiteren Schritt c) mit Glykol oder 1,3 Propandiol (Buehler et al., Nucleosides, Nucleotides & Nucleic Acids 24 (2005) 891) zum Schutz der Carbonylgruppe ketalisiert werden. Die daraus resultierenden Dioxolane bzw. Dioxane führen durch Umsetzung mit Triton-B / Paraformaldehyd (Buehler et al., Nucleosides, Nucleotides & Nucleic Acids 24 (2005) 891) und anschließender Entschützung mit HCl / Wasser (Buehler et al., Nucleosides, Nucleotides & Nucleic Acids 24 (2005) 891) zu [3-(2-Hydroxy-1-methyl-ethyl)- 4-nitrophenyl]-aryl-methanonen.

Das entstandene [3-(2-Hydroxy-1-methyl-ethyl)-4-nitro-phenyl]-aryl-methanon kann als Ausgangsstoff für die Synthese eines Nukleosids enthaltend eine photolabile NPPOC-Schutzgruppe eingesetzt werden (Buehler et al., Nucleosides, Nucleotides & Nucleic Acids 24 (2005) 891; Helv. Chim. Acta 87 (2004) 620; WO 2004/074300). Das entsprechende Nukleosid kann dann in ein Phosphoramidit mit photolabiler NPPOC-Schutzgruppe umgewandelt werden (WO 2004/074300).

Gegenstand der vorliegenden Erfindung sind auch die Substanzen (3-Ethyl-4-nitrophenyl)-aryl-methanon oder [3-(2-Hydroxy-1-methyl-ethyl)- 4-nitrophenyl]-aryl-methanon, dadurch gekennzeichnet, dass das Aryl Naphthalin ist.

Gegenstand der vorliegenden Erfindung sind auch Nukleoside, die ein [3-(2-O-1-methyl-ethyl)-4-nitrophenyl]-aryl-methanon substituiert an der 5' oder 3' Position enthalten. Vorzugsweise ist dieser Substituent über einen O-Carbonat-Ester an das Nukleosid gekoppelt. Ebenfalls bevorzugt sind entsprechende NukleosidPhosphoramidite, bei denen sich die Phosphoramiditgruppe an derjenigen 3' oder 5' Position befindet, an der kein [3-(2-O-1-methyl-ethyl)-4-nitrophenyl] -arylmethanon substituiert ist.

### Abbildungen

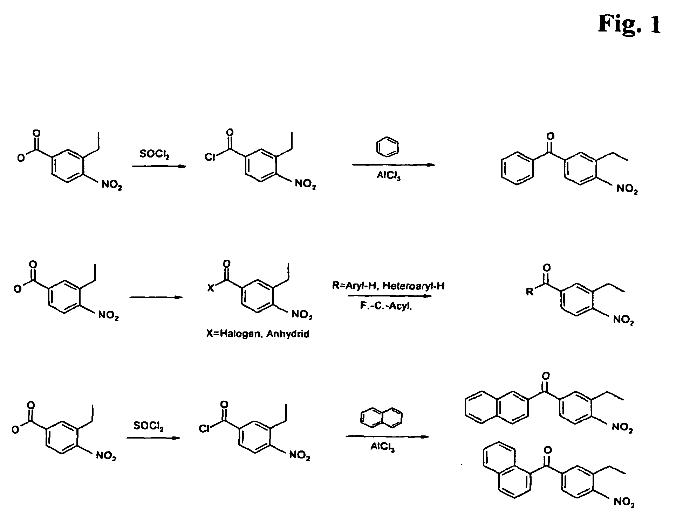

- **Fig. 1.:**: Schematische Darstellung von erfindungsgemäßen Synthesewegen in allgemeiner Form (mittlere Zeile) sowie für die spezielle Reaktion mit Benzol (obere Zeile) bzw. Naphthalin (untere Zeile).

### Detaillierte Beschreibung der Erfindung

Die vorliegende Erfindung betrifft ein neues Verfahren zur Herstellung von Vorstufen von photolabilen NPPOC Schutzgruppen., wie beispielsweise (3-Ethyl-4-nitrophenyl)-phenyl-methanon, welches in der Fachwelt auch als 3-Ethyl-4-nitro-benzophenon bezeichnet wird. Das erfindungsgemäße Verfahren ermöglicht darüber hinaus die Herstellung von bisher nicht synthetisch zugänglichen Vorstufen, aus denen bisher unbekannte NPPOC Derivate herstellbar sind.

Das erfindungsgemäße Verfahren zur Herstellung von Schutzgruppen des NPPOC Typs beruht im Wesentlichen auf zwei aufeinander folgenden Schritten. Im ersten Schritt erfolgt eine Halogenierung und vorzugsweise eine Chlorierung von kommerziell erhältlicher 3-Ethyl-4-nitrobenzoesäure, wobei die bisher aus dem Stand der Technik nicht bekannte Zwischenverbindung 3-Ethyl-4-nitrobenzoesäurechlorid entsteht.

In einem zweiten Schritt erfolgt dann durch Friedel-Crafts-Acylierung eine Substitution des Chloratoms durch ein beliebiges gegebenenfalls substituiertes Aryl, welches mindestens noch ein H-Atom aufweist, so dass dieses im Folgenden als Aryl-H bezeichnet wird. Dabei entsteht (3-Ethyl-4-nitrophenyl)-aryl-methanon.

Wie aus den Beispielen ersichtlich, kann das erfindungsgemäße Herstellverfahren im Eintopf-Verfahren mit 85% Ausbeute problemlos und technisierbar in größerem Maßstab durchgeführt werden.

Handelt es sich bei dem Aryl um Benzol, so entsteht 3-Ethyl-4-nitro-benzophenon, bzw. gemäß IUPAC Nomenklatur (3-Ethyl-4-nitrophenyl)-phenyl-methanon.

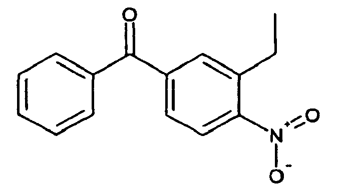

Das entsprechende Syntheseverfahren ist in der oberen Zeile von Fig. 1 schematisch dargestellt. Kommerziell erhältliche 3-Ethyl-4-nitrobenzoesäure wird im ersten Schritt durch Umsetzung mit Thionylchlorid zu 3-Ethyl-4-nitrobenzoesäurechlorid umgesetzt. Dann erfolgt die Friedel-Crafts-Acylierung mit Benzol in Gegenwart von Aluminiumchlorid.

Eine allgemeine Form des erfindungsgemäßen Syntheseverfahrens findet sich in der mittleren Zeile von Fig. 1. Ausgangspunkt der Synthese ist jeweils 3-Ethyl-4-nitrobenzoesäure, welche im ersten Schritt durch geeignete Verfahren zum entsprechenden Säurehalogenid umgesetzt wird und anschließend mit Hilfe einer Friedel-Crafts-Acylierung mit einem beliebigen Aryl acyliert werden kann. Das jeweils verwendete Aryl kann bis auf eine Position in beliebiger Weise substituiert sein.

Anstelle der Umsetzung von 3-Ethyl-4-nitrobenzoesäure mit einem Thionylhalogenid kann als erster Syntheseschritt aus 3-Ethyl-4-nitrobenzoesäure durch Entzug von Wasser ein 3-Ethyl-4-nitrobenzoesäureanhydrid hergestellt werden. Derartige Anhydride sind ebenfalls einer Friedel-Crafts-Acylierung in analoger Weise zugänglich.

Da das erfindungsgemäße Verfahren eine allgemeine Syntheseroute zu den bisher nicht synthetisch zugänglichen Stoffen der Aryl- bzw. Hetero-Analoga des 3-Ethyl-4-nitro-benzophenons aufzeigt, bezieht sich ein Aspekt der Erfindung auch auf spezielle Substanzen enthaltend eine (3-Ethyl-4-nitrophenyl)-aryl-methanon Struktur, welche dadurch gekennzeichnet ist, dass der gegebenenfalls substituierte Arylrest ein gegebenenfalls substituierter kondensierter Aromat ist.

Dabei kann der kondensierte Aromat aus 2-5 beliebigen homozyklischen oder heterozyklischen Ringsystemen bestehen, wobei jeder Ring unabhängig voneinander entweder einen Hexazyklus oder einen Pentazyklus bildet.

Eine besonders bevorzugte Ausführungsformen stellt dabei (3-Ethyl-4-nitrophenyl)-naphthalen-1-yl-methanon dar:

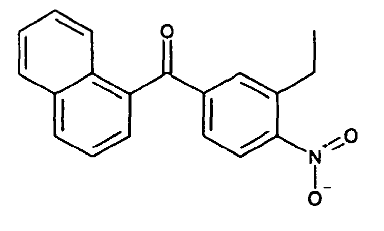

Die Synthese ist in der unteren Zeile von Fig. 1 schematisch dargestellt. Dabei können bei der Friedel-Crafts-Acylierung von 3-Ethyl-4-nitrobenzoesäurechlorid mit Naphthalin mit (3-Ethyl-4-nitro-phenyl)-naphthalen-1-yl-methanon bzw. (3-Ethyl-4-nitro-phenyl)-naphthalen-2-yl-methanon prinzipiell zwei verschiedene Stellungsisomere entstehen, wobei die bevorzugte Entstehung von (3-Ethyl-4-nitrophenyl)-naphthalen-1-yl-methanon dadurch gesteuert werden kann, dass die Acylierung bei möglichst tiefen Temperaturen durchgeführt wird. Eine Isolierung des 1-Isomers erfolgt dabei bevorzugt durch entsprechende, dem Fachmann bekannte Kristallisationsverfahren. (3-Ethyl-4-nitro-phenyl)-naphthalen-2-yl-methanon entsteht dagegen bevorzugt bei höheren Temperaturen. Eine Isolierung des 2-Isomers erfolgt in diesem Falle bevorzugt durch dem Fachmann bekannte Säulenchromatographische Verfahren.

Darüber hinaus können die erfindungsgemäß entstandenen (3-Ethyl-4-nitrophenyl)-aryl-methanone mithilfe geeigneter Syntheseverfahren zu [3-(2-Hydroxy-1-methyl-ethyl)- 4-nitrophenyl]-aryl-methanonen umgewandelt werden (Buehler et al., Nucleosides, Nucleotides & Nucleic Acids 24 (2005) 891; Helv. Chim. Acta 87 (2004) 620; WO 2004/074300). In einem ersten Schritt erfolgt die Umsetzung mit geeigneten Diolen zur Erzeugung von Dioxolanen oder Dioxanen, welche zunächst mit Triton B / Paraformaldehyd und anschließend HCl / Wasser versetzt werden (Buehler et al., Nucleosides, Nucleotides & Nucleic Acids 24 (2005) 891; Helv. Chim. Acta 87 (2004) 620; WO 2004/074300).

Dies wird im Folgenden am Beispiel des (3-Ethyl-4-nitro-phenyl)-naphthalen-1-yl-methanons näher erläutert.

In einer ersten Ausführungsform wird beispielsweise (3-Ethyl-4-nitro-phenyl)-naphthalen-1-yl-methanon zunächst mit Glykol versetzt. Dabei entsteht 2-(3-Ethyl-4-nitro-phenyl)-2-naphtalen-1-yl-[1,3] dioxolane.

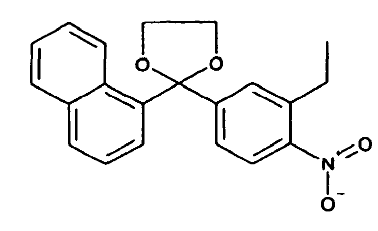

Anschließend wird das 2-(3-Ethyl-4-nitro-phenyl)-2-naphtalen-1-yl-[1,3] dioxolane in Gegenwart von Triton B / Paraformaldehyd zum 2-[5-(2-Naphthalen-1-yl-[1,3]dioxolan-2-yl)-2-nitro-phenyl]-propan-1-ol hydroxymethyliert.

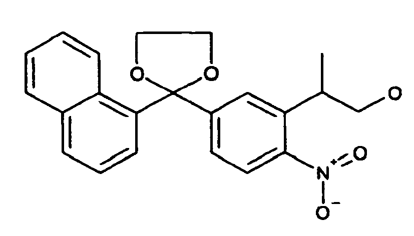

Nach Behandlung mit HCl / Wasser entsteht schließlich [3-(2-Hydroxy-1-methyl-ethyl)-4-nitro-phenyl]-naphthalen-1-yl-methanon.

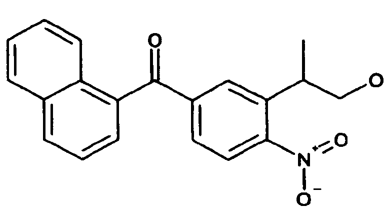

In einer alternativen Ausführungsform wird beispielsweise (3-Ethyl-4-nitrophenyl)-naphthalen-1-yl-methanon zunächst mit 1,3 Propandiol versetzt. Dabei entsteht 2-(3-Ethyl-4-nitro-phenyl)-2-naphtalen-1-yl-[1,3] dioxan.

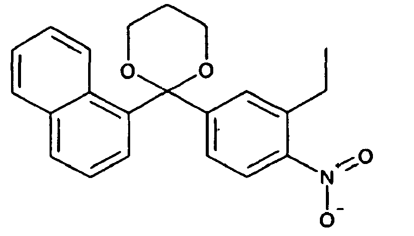

Anschließend wird das 2-(3-Ethyl-4-nitro-phenyl)-2-naphtalen-1-yl-[1,3] dioxan ebenfalls in Gegenwart von Triton B / Paraformaldehyd zum (2-[5-(2-Naphthalen-1-yl-[1,3]dioxan-2-yl)-2-nitro-phenyl]-propan-1-ol hydroxymethyliert.

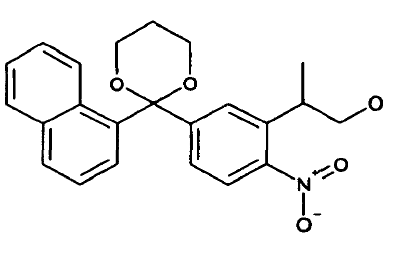

Nach Behandlung mit HCl / Wasser entsteht dann ebenfalls [3-(2-Hydroxy-1-methyl-ethyl)-4-nitro-phenyl]-naphthalen-1-yl-methanon.

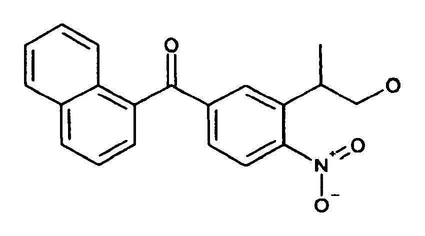

Die mithilfe der oben beschriebenen Synthese entstandenen erfindungsgemäßen Substanzen stellen photolabile Schutzgruppen bzw. Vorläufer der sogenannten NPPOC Klasse dar, die durch ein 2-(2-Nitrophenyl) ethyl Grundgerüst gekennzeichnet sind. Diese Struktur kann an Nukleoside gekoppelt und anschließend in Nukleosid-Phosphoramidite umgewandelt werden. Derartige Phosphoramidite enthalten somit Schutzgruppen die durch Photolyse abspaltbar sind.

Gegenstand der vorliegenden Erfindung sind daher auch Nukleoside sowie Nukleosidphosphoramidite, die (3-Ethyl-4-nitrophenyl)-aryl-methanon bzw. [3-(2-Hydroxy-1-methyl-ethyl)-4-nitrophenyl]-aryl-methanon Schutzgruppen enthalten, welche dadurch gekennzeichnet sind, dass das Aryl Naphthalin ist. Besonders bevorzugt handelt es sich dabei um Phosphoramidite enthaltend (3-Ethyl-4-nitrophenyl)-naphthalen-1-yl-methanon bzw. [3-(2-O-1-methyl-ethyl)-4-nitro-phenyl]-naphthalen-1-yl-methanon.

Die Herstellung von Nukleosiden und Nukleosid-Phosphoramiditen enthaltend die erfindungsgemäßen photolabilen Schutzgruppen erfolgt nach dem Fachmann bekannten Standardkopplungsmethoden wie sie beispielsweise in (Buehler et al., Nucleosides, Nucleotides & Nucleic Acids 24 (2005) 891; Helv. Chim. Acta 87 (2004) 620; WO 2004/074300; WO 97/44345) beschrieben sind. Dabei wird zunächst der Alkohol [3-(2-Hydroxy-1-methyl-ethyl)- 4-nitrophenyl]-aryl-methanon mithilfe von Phosgen oder dessen Abkömmlingen wie beispielsweise Diphosgen oder Triphosgen (WO 2004/074300) in den entsprechenden Chlorameisensäureester überführt.

Anschließend erfolgt die Kopplung an das jeweilige Nukleosid oder Nukleosid-Derivat. Vorzugsweise werden daraus Phosphoramidit-Nukleosid Derivate hergestellt, da die im Folgenden entstehenden und mit einer Schutzgruppe versehenen Derivate direkt als Bausteine für die konventionelle Oligonukleotidsynthese einsetzbar sind.

Phosphoramidit-Nukleosid Derivate können nach dem Fachmann bekannten Verfahren durch Umsetzung von Nukleosiden mit Phosphanen (frühere Nomenklatur: Phosphine) in Gegenwart von Tetrazol erzeugt werden (Buehler et al., Nucleosides, Nucleotides & Nucleic Acids 24 (2005) 891; WO 2004/074300). In der Regel werden 3' Phosphoramidite erzeugt, da diese für die konventionelle Oligonukleotidsynthese in 3' - 5' Orientierung verwendet werden können. Alternativ werden 5' Phosphoramidite erzeugt, die für eine inverse Oligonukleotidsynthese verwendet werden können.

Die hergestellten Chlorameisensäureester können mit der freien Hydroxylgruppe von Nukleosiden oder Nukleosid-Derivaten reagieren, so dass deren Kohlensäure - 2-[5-(arylene carbonyl)-2-nitro-phenyl]-propylester bzw. deren Phosphoramidit-Derivate entstehen. Letztere sind dann unmittelbar als photoaktivierbare Bausteine in der Oligonukleotidsynthese einsetzbar.

### Beispiele

### Beispiel 1

### (3-Ethyl-4-nitro-phenyl)-phenyl-methanon (= 3-Ethyl-4-nitro-benzophenon):

7 g (36 mmol) 3-Ethyl-4-nitro-benzoesäure wurden in 15 ml (205 mmol) Thionylchlorid 30 min unter Rühren am Rückfluss gekocht (bis keine Gasentwicklung mehr auftrat). Danach wurde bei 50°C überschüssiges Thionylchlorid unter Vakuum abdestilliert, der Eindampfrückstand in 20 ml (225 mmol) Benzol gelöst und portionsweise mit 6,5 g (49 mmol) Aluminiumchlorid versetzt. Das Gemisch wurde 2,5 h unter Rühren am Rückfluss gekocht, anschließend auf Raumtemperatur abgekühlt und in 75 g Eiswasser eingegossen. Die wässrige Phase wurde zweimal mit je 25 ml Essigsäureethylester extrahiert, die organischen Phasen aufkonzentriert und der Rückstand aus Ethanol mit A-Kohle umkristallisiert. Ausbeute: 7,9 g (85% d. Th.); hellgelbe Kristalle; Fp: 64-65°C; Reinheit: 99% (HPLC); NMR und Massenspektroskopie: entspricht.

### Beispiel 2

### (3-Ethyl-4-nitro-phenyl)-phenyl-methanon ( = 3-Ethyl-4-nitro-benzophenon):

35 g (0,18 mol) 3-Ethyl-4-nitro-benzoesäure wurden in 75 ml (1,03 mol) Thionylchlorid 30 min unter Rühren am Rückfluss gekocht (bis keine Gasentwicklung mehr auftrat). Danach wurde bei 50°C überschüssiges Thionylchlorid unter Vakuum abdestilliert, der Eindampfrückstand in 50 ml (0,57 mol) Benzol gelöst und innerhalb von 10 min zu einer Mischung aus 32,5 g (0,25 mol) Aluminiumchlorid in 50 ml (0,57 mol) Benzol getropft. Das Gemisch wurde 2,5 h unter Rühren am Rückfluss gekocht, anschließend auf Raumtemperatur abgekühlt und in 375 g Eiswasser eingegossen. Die wässrige Phase wurde mit 10 ml konz. HCl versetzt, anschließend zweimal mit je 150 ml Essigsäureethylester extrahiert, die organischen Phasen zweimal mit je 75 ml Wasser gewaschen, aufkonzentriert und der Rückstand aus Ethanol mit A-Kohle umkristallisiert. Ausbeute: 36,7 g (80% d. Th.); hellgelbe Kristalle; Fp: 63-64°C.

### Beispiel 3

### (3-Ethyl-4-nitro-phenyl)-naphthalen-1-yl-methanon:

7,0 g (36 mmol) 3-Ethyl-4-nitro-benzoesäure wurden in 15 ml (205 mmol) Thionylchlorid 30 min unter Rühren am Rückfluss gekocht (bis keine Gasentwicklung mehr auftrat) und danach wurde bei 50°C überschüssiges Thionylchlorid unter Vakuum abdestilliert. Der Eindampfrückstand und 4,5 g (35 mmol) Naphthalin wurden in 35 ml Dichlormethan gelöst, auf - 40°C abgekühlt und innerhalb 1 Stunde portionsweise mit 6,5 g (49 mmol) Aluminiumchlorid versetzt. Das Gemisch wurde 30 min bei -40°C, 2 h bei -20°C gerührt, in 300 g Eiswasser eingegossen und mit 80 ml Dichlormethan extrahiert. Die organische Phase wurde mit 100 ml Wasser und mit 100 ml gesättigter Natriumhydrogencarbonat-Lösung gewaschen, aufkonzentriert und der Rückstand aus Ethanol umkristallisiert. Ausbeute: 7,7 g (70% d. Th.); hellgelbe Kristalle; Fp: 57-58°C; Reinheit: 99% (HPLC); NMR und Massenspektroskopie: entspricht.

### Beispiel 4

### 2-(3-Ethyl-4-nitro-phenyl)-2-naphtalen-1-yl-[1,3] dioxolane:

7,6 g (25 mmol) (3-Ethyl-4-nitro-phenyl)-naphthalen-1-yl-methanon, 1,2 g (6 mmol) p-Toluolsulfonsäure und 29 ml (520 mmol) Glykol in 41 ml Toluol wurden 24 h unter Rühren am Wasserabscheider gekocht. Anschließend wurde mit 17 ml 2% Natronlauge und zweimal mit je 22 ml gesättigter Kochsalz-Lösung gewaschen, aufkonzentriert und der Rückstand aus Methanol umkristallisiert. Ausbeute: 7,6 g (87% d. Th.); hellgelbe Kristalle; Fp: 88-90°C; NMR und Massenspektroskopie: entspricht.

### Beispiel 5

### 2-[5-(2-Naphthalen-1-yl-[1,3]dioxolan-2-yl)-2-nitro-phenyl]-propan-1-ol:

7,0 g (20 mmol)2-(3-Ethyl-4-nitro-phenyl)-2-naphtalen-1-yl-[1,3] dioxolane, 2,4 g (27 mmol) Paraformaldehyd und 30 ml DMSO wurden mit 6,4 ml Triton B (35% in Methanol) 3 h bei 90°C gerührt. Anschließend wurde mit 35 ml Dichlormethan und 60 ml Wasser versetzt, extrahiert und die organische Phase zweimal mit je 40 ml Wasser gewaschen, aufkonzentriert und der Rückstand aus Diisopropylether umkristallisiert. Ausbeute: 7,0 g (92% d. Th.); hellgelbe Kristalle; Fp: 115°C; NMR und Massenspektroskopie: entspricht.

### Beispiel 6

### [3-(2-Hydroxy-1-methyl-ethyl)- 4-nitro-phenyl]-naphthalen-l-yl-methanon:

6,8 g (18 mmol) 2-[5-(2-Naphthalen-1-yl-[1,3]dioxolan-2-yl)-2-nitro-phenyl]-propan-1-ol gelöst in 22 ml Ethanol wurden mit 4,3 ml konz. HCl versetzt und 2,5 h unter Rühren am Rückfluss gekocht. Anschließend wurde mit 25 ml Dichlormethan und 50 ml Wasser versetzt, extrahiert und die organische Phase zweimal mit je 38 ml Wasser gewaschen, getrocknet und das Lösungsmittel unter Vakuum entfernt. Ausbeute: 6 g (quant.); zähes gelbes Öl; NMR und Massenspektroskopie: entspricht.

## Patentansprüche

1. Syntheseverfahren enthaltend die folgenden Schritte
a) Umsetzung von 3-Ethyl-4-nitrobenzoesäure mit einem Thionylhalogenid zur Erzeugung eines 3-Ethyl-4-nitrobenzoesäurehalogenids
b) Friedel-Crafts-Acylierung durch Reaktion des 3-Ethyl-4-nitrobenzoesäurehalogenids oder des 3-Ethyl-4-nitrobenzoesäureanhydrids mit gegebenenfalls substituiertem Aryl-H, so dass ein gegebenenfalls substituiertes (3-Ethyl-4-nitrophenyl)-aryl-methanon entsteht.

2. Verfahren nach Anspruch 1, **dadurch gekennzeichnet, dass** 3-Ethyl-4-nitrobenzoesäure mit Thionylchlorid umgesetzt wird.

3. Verfahren nach Anspruch 1-2, **dadurch gekennzeichnet, dass** Aryl-H Benzol ist.

4. Verfahren nach Anspruch 1-2, **dadurch gekennzeichnet, dass** Aryl-H ein gegebenenfalls substituierter kondensierter Aromat ist.

5. Verfahren nach Anspruch 4, **dadurch gekennzeichnet, dass** Aryl-H Naphthalin ist.

6. Verfahren nach Anspruch 1-5, **dadurch gekennzeichnet, dass** das entstandene Produkt in einem weiteren Schritt c) mit Glykol oder 1,3 Propandiol umgesetzt wird.

7. Verfahren nach Anspruch 6, **dadurch gekennzeichnet**, das das in Schritt c) entstandene Produkt in einem weiteren Schritt d) zunächst mit Triton B / Paraformaldehyd und anschießend mit HCl / Wasser zu [3-(2-Hydroxy-1-methyl-ethyl)-4-nitro-phenyl]-aryl-methanon umgewandelt wird.

8. Verfahren nach Anspruch 1-7, **dadurch gekennzeichnet, dass** das entstandene [3-(2-Hydroxy-1-methyl-ethyl)-4-nitro-phenyl]-aryl-methanon als Ausgangsstoff für die Synthese eines Nukleosids enthaltend eine photolabile Schutzgruppe eingesetzt wird.

9. Verfahren nach Anspruch 8, **dadurch gekennzeichnet, dass** das Nukleosid enthaltend eine photolabile Schutzgruppe in ein Phosphoramidit umgewandelt wird.

10. (3-Ethyl-4-nitrophenyl)-aryl-methanon oder [3-(2-Hydroxy-1-methyl-ethyl)-4-nitrophenyl]-aryl-methanon, **dadurch gekennzeichnet, dass** das Aryl Naphtalin ist.

11. Nukleosid, enthaltend einen [3-(2-O-1-methyl-ethyl)-4-nitrophenyl]-naphthalin-methanon Substituenten an der 5' oder 3' Position, **dadurch gekennzeichnet, dass** der Substituent über eine Carbonatestergruppe an das Nukleosid gekoppelt ist.

12. Nukleosid nach Anspruch 11, welches eine Phosphoramiditgruppe aufweist, die sich an derjenigen freien 3' oder 5' Position befindet, welche keinen [3-(2-O-1-methyl-ethyl)-4-nitrophenyl]-aryl-methanon Substituenten enthält.

## Claims

1. Synthetic method comprising the following steps
a) reaction of 3-ethyl-4-nitrobenzoic acid with a thionyl halide to produce a 3-ethyl-4-nitrobenzoyl halide
b) Friedel-Crafts acylation by reaction of the 3-ethyl-4-nitrobenzoyl halide or the 3-ethyl-4-nitrobenzoic anhydride with optionally substituted aryl-H to form an optionally substituted (3-ethyl-4-nitrophenyl)aryl-methanone.

2. Method according to Claim 1, **characterised in that** 3-ethyl-4-nitrobenzoic acid is reacted with thionyl chloride.

3. Method according to Claims 1-2, **characterised in that** aryl-H is benzene.

4. Method according to Claims 1-2, **characterised in that** aryl-H is an optionally substituted fused aromatic compound.

5. Method according to Claim 4, **characterised in that** aryl-H is naphthalene.

6. Method according to Claims 1-5, **characterised in that** the product formed is reacted in a further step c) with glycol or propane-1,3-diol.

7. Method according to Claim 6, **characterised in that** the product formed in step c) is converted in a further step d), firstly with Triton B/paraformaldehyde and subsequently with HCl/water, to give [3-(2-hydroxy-1-methylethyl)-4-nitrophenyl]arylmethanone.

8. Method according to Claims 1-7, **characterised in that** the [3-(2-hydroxy-1-methylethyl)-4-nitrophenyl]arylmethanone formed is used as the starting material for the synthesis of a nucleoside comprising a photolabile protecting group.

9. Method according to Claim 8, **characterised in that** the nucleoside comprising a photolabile protecting group is converted into a phosphoramidite.

10. (3-Ethyl-4-nitrophenyl)arylmethanone or [3-(2-hydroxy-1-methylethyl)-4-nitrophenyl]aryl-methanone, **characterised in that** the aryl is naphthalene.

11. Nucleoside comprising a [3-(2-0-1-methylethyl)-4-nitrophenyl]naphthalenemethanone substituent at the 5' or 3' position, **characterised in that** the substituent is linked to the nucleoside by a carbonate ester group.

12. Nucleoside according to Claim 11, having a phosphoramidite group which is located at that free 3' or 5' position which comprises no [3-(2-O-1-methylethyl)-4-nitrophenyl]arylmethanone substituent.

## Revendications

1. Procédé de synthèse contenant les étapes suivantes
a) transformation d'un acide 3-éthyl-4-nitrobenzoïque avec un halogénure de thionyle pour générer un halogénure de l'acide 3-éthyl-4-nitrobenzoïque
b) acylation de Friedel-Crafts par réaction du chlorure de 3-éthyl-4-nitrobenzoïque ou de l'anhydride de l'acide 3-éthyl-4-nitrobenzoïque avec aryl-H le cas échéant substitué, de manière telle qu'on obtient une (3-éthyl-4-nitrophényl)-arylméthanone.

2. Procédé selon la revendication 1, **caractérisé en ce que** l'acide 3-éthyl-4-nitrobenzoïque est transformé avec du chlorure de thionyle.

3. Procédé selon la revendication 1-2, **caractérisé en ce que** aryl-H est le benzène.

4. Procédé selon la revendication 1-2, **caractérisé en ce que** aryl-H est un aromatique condensé le cas échéant substitué.

5. Procédé selon la revendication 4, **caractérisé en ce que** aryl-H est le naphtalène.

6. Procédé selon la revendication 1-5, **caractérisé en ce que** le produit formé est transformé dans une étape c) ultérieure avec du glycol ou du 1,3-propanediol.

7. Procédé selon la revendication 6, **caractérisé en ce que** le produit formé dans l'étape c) est transformé, dans une étape d) ultérieure, d'abord avec un mélange Triton B/paraformaldéhyde et ensuite avec un mélange HCl/eau en [3-(2-hydroxy-1-méthyléthyl)-4-nitrophényl]-arylméthanone.

8. Procédé selon la revendication 1-7, **caractérisé en ce que** la [3-(2-hydroxy-1-méthyléthyl)-4-nitrophényl]-arylméthanone est utilisée comme substance de départ pour la synthèse d'un nucléoside contenant un groupe de protection photolabile.

9. Procédé selon la revendication 8, **caractérisé en ce que** le nucléoside contenant un groupe de protection photolabile est transformé en un phosphoramidite.

10. (3-éthyl-4-nitrophényl)-arylméthanone ou [3-(2-hydroxy-1-méthyléthyl)- 4-nitrophényl]-arylméthanone, **caractérisée en ce que** aryle est le naphtalène.

11. Nucléoside contenant un substituant [3-(2-O-1-méthyléthyl)-4-nitrophényl]-naphtalène-méthanone en position 5' ou 3', **caractérisé en ce que** le substituant est couplé via un groupe carbonate-ester au nucléoside.

12. Nucléoside selon la revendication 1, présentant un groupe phosphoramidite, qui se trouve en la position 3' ou 5' libre qui ne contient pas de substituant [3-(2-O-1-méthyléthyl)-4-nitrophényl]-arylméthanone.
